**Europäisches Patentamt**

**European Patent Office**

**Office européen des brevets**

(11) Veröffentlichungsnummer: **0 369 392**
**A2**

(12) **EUROPÄISCHE PATENTANMELDUNG**

(21) Anmeldenummer: **89121047.8**

(22) Anmeldetag: **14.11.89**

(30) Priorität: **18.11.88 DE 3838982**

(43) Veröffentlichungstag der Anmeldung:
**23.05.90 Patentblatt 90/21**

(84) Benannte Vertragsstaaten:
**ES GR**

(51) Int. Cl.⁵: **A61F 5/48**

(71) Anmelder: **Akzo N.V.**
**Postbus 9300 Velperweg 76**
**NL-6800 SB Arnhem(NL)**

(72) Erfinder: **Nieveld-Molenaar, Elisabeth**
**Petronella**
**Lijsterstraat 32.2**
**NL-2042 CJ Zandvoort(NL)**

(74) Vertreter: **Fett, Günter**
**Akzo Patente GmbH Kasinostrasse 19 - 23**
**D-5600 Wuppertal 1(DE)**

(54) **Saugfähige Matratzenauflage.**

(57) Saugfähige Matratzenauflage, bestehend aus wenigstens zwei Schichten, von denen die erste, der Matratze zugewandte Schicht im wesentlichen flüssigkeitsundurchlässig ist und die darüberliegende zweite Schicht als flüssigkeitsaufnehmende Sauglage ausgebildet ist, wobei die im wesentlichen flüssigkeitsundurchlässige Schicht als Folie mit hoher Wasserdampfdurchlässigkeit ausgebildet ist.

EP 0 369 392 A2

## Saugfähige Matratzenauflage

Die Erfindung betrifft eine saugfähige Matratzenauflage, bestehend aus wenigstens zwei Schichten, von denen die erste, der Matratze zugewandte Schicht im wesentlichen flüssigkeitsundurchlässig ist und die darüberliegende zweite Schicht als flüssigkeitsaufnehmende Sauglage ausgebildet ist.

Derartige Matratzenauflagen werden zum Schutz von Matratzen bei solchen Patienten verwendet, die unter der sog. Inkontinenz für Harnleiden, einer Erkrankung, die temporär oder auch bleibend auftreten kann.

Eine Matratzenauflage der eingangs genannten Art besteht im einfachsten Fall aus einem flüssigkeitsundurchlässigen Flächengebilde, wie z.B. einem gummierten Tuch und einer darüberliegend angeordneten Sauglage, die ein hohes Maß an Feuchtigkeitsaufnahmevermögen besitzt.

Der Nachteil einer solchen Matratzenauflage besteht darin, daß der ausgeschiedene Harn praktisch vollständig in der Sauglage verbleibt. Dies führt zu einem unangenehmen Kältegefühl an den im Bereich der Sauglage befindlichen Körperteilen. Darüberhinaus werden bei länger anhaltenden Krankheitszuständen häufig Reizungen der betroffenen Hautpartien beobachtet, die in vielen Fällen schwere Hautschäden nach sich ziehen können. Besonders bei bettlägerigen Patienten, die z.B. aufgrund einer Schädigung des Rückenmarks den Abgang des Harns nicht bemerken, ist eine häufige Kontrolle der Sauglage erforderlich.

Aufgabe der Erfindung war es daher, eine Matratzenauflage der eingangs erwähnten Art zur Verfügung zu stellen, bei der die geschilderten Probleme nicht mehr auftreten. Insbesondere soll erreicht werden, daß die Patienten nur verhältnismäßig kurze Zeit der Feuchtigkeit ausgesetzt sind, ohne daß die Matratzenauflage bzw. die Sauglage jeweils nach dem Abgang von Harn gewechselt werden müssen.

Diese Aufgabe wird erfindungsgemäß durch eine gattungsgemäße Matratzenauflage mit den kennzeichnenden Merkmalen des Anspruchs 1 gelöst.

Bei der erfindungsgemäßen Matratzenauflage ist die im wesentlichen flüssigkeitsundurchlässige Schicht als Folie mit hoher Wasserdampfdurchlässigkeit ausgebildet. Diese bewirkt, daß die in der Sauglage nach dem Abgang von Harn vorhandene Flüssigkeit durch die als Membran wirkende Folie in dampfförmiger Phase durch die Matratze hindurch an die Umgebung abgeführt wird. Die darüberliegende Sauglage muß deshalb den Harn nur vorübergehend aufnehmen und ist nach erfolgtem Abtransport der Flüssigkeit wieder in trockenem Zustand. Damit entfällt die Notwendigkeit zu einem häufigen Wechsel der Sauglage, da der Patient bereits innerhalb relativ kurzer Zeit trocken liegt. Die oben beschriebenen feuchtigkeitsbedingten Hautreizungen und -schädigungen werden nicht mehr beobachtet. Außerdem wird das Wohlbefinden der Patienten wesentlich gesteigert, da das unangnehme Kältegefühl nicht mehr auftritt.

Bei der Auswahl einer derartigen Folie ist darauf zu achten, daß sie zum einen eine hohe Wasserdampfdurchlässigkeit aufweist, die erforderlich ist, um die Flüssigkeit möglichst rasch abzuführen. Für die erfindungsgemäße Matratzenauflage hat sich eine Wasserdampfdurchlässigkeit von mindestens 500 g/m$^2$/Tag/23°C/100-50% relative Luftfeuchtigkeit bei einer Windgeschwindigkeit von 0,3 m/s als geeignet erwiesen.

Zur Bestimmung der Wasserdampfdurchlässigkeit wird folgendes Verfahren angewendet:

In eine Zelle mit einem Durchmesser von 5,6 cm werden 3 g eines Polymers mit einem sehr hohen Wasseraufnahmevermögen ("Superslurper"), das mit 20 g Wasser gesättigt wurde, eingebracht. Die zu untersuchende Folie wird dann so über das Polymer gespannt, daß sie durch dieses gut angefeuchtet wird. Die Gewichtsabnahme pro Stunde bei einer Temperatur von 23°C, einer relativen Luftfeuchtigkeit von 50% und einer Windgeschwindigkeit von 0,3 m/s ist ein Maß für die Wasserdampfdurchlässigkeit.

Andererseits ist zu beachten, daß die Folie eine ausreichende Dichtheit aufweist, um die Matratze sicher zu schützen. Sie kann als gegeben betrachtet werden, wenn der Wert der Flüssigkeitsdurchlässigkeit maximal 0,02 g/cm$^2$/Std/23°C/0,07 bar beträgt.

Der Wert der Flüssigkeitsdurchlässigkeit wird nach folgender Vorschrift bestimmt:

Eine kreisförmige Probe der zu untersuchenden Folie mit einem Durchmesser von 6,15 cm wird zwischen eine wasseraufnehmende Schicht und ein Filterpapier mit den gleichen Abmessungen gelegt. Als wasseraufnehmende Schicht verwendet man 1 g eines Polymers mit einem sehr hohen Wasseraufnahmevermögen, das mit 5 g Wasser gesättigt wurde und sich zwischen zwei Papiertüchern befindet. Das sich nicht an der Folienseite befindende Papiertuch ist mit einer Lage eines hydrophoben Vliesstoffes abgedeckt.

Die Gewichtszunahme des Filterpapiers nach 5 Minuten unter Einwirkung eines Gewichtes von 2 kg, das an der Seite des Vliesstoffes gleichmäßig über das zu untersuchende Produkt verteilt ist, ist ein Maß für die Wasserdichtheit.

Eine Folie, die beide Bedingungen erfüllt, ist beispielsweise in der Europäischen Patentanmeldung mit dem Aktenzeichen 88 116 748.0 beschrieben. Sie ist aus einem Copolyetherester hergestellt, der aus einer Vielzahl sich wiederholender intralinearer Estergruppen mit einem langen Kettenmolekül und Estergruppen mit einem kurzen Kettenmolekül besteht, die durch Esterbindungen auf statistische Weise so miteinander verbunden sind, daß der Anfang und das Ende jeweils aneinander angrenzen, wobei die Estergruppen mit langkettigen Estereinheiten der folgenden Formel entsprechen:

$$-O\,G\,O\,\overset{\overset{\displaystyle O}{\|}}{C}\,R\,\overset{\overset{\displaystyle O}{\|}}{C}-$$

wobei die Estergruppen mit kurzkettigen Estereinheiten der Formel entsprechen:

$$-O\,D\,O\,\overset{\overset{\displaystyle O}{\|}}{C}\,R\,\overset{\overset{\displaystyle O}{\|}}{C}-$$

wobei G einen zweiwertigen Rest darstellt, die nach Entfernung von endständigen Hydroxylgruppen von mindestens einem Glycol mit einem langen Kettenmolekül, einem Molekulargewicht von mehr als 800 und einem mittleren Atomverhältnis von Kohlenstoff zu Sauerstoff von 2,0 übrigbleibt, wobei R einen zweiwertigen Rest darstellt, der nach Entfernung von Carboxylgruppen von mindestens einer Carbonsäure mit einem Molekulargewicht von weniger als 300 übrigbleibt, und D einen zweiwertigen Rest darstellt, der nach Entfernung von Hydroxylgruppen von mindestens einem Diol mit einem Molekulargewicht von weniger als 250 übrigbleibt, wobei die verwendete Dicarbonsäure zu mindestens 75 Mol% aus Terephthalsäure oder deren esterbildenden Äquivalenten besteht und das Diol mit einem niedrigen Molekulargewicht zu mindestens 75 Mol% aus 1,4-Butandiol oder dessen esterbildenden Äquivalenten besteht, die Summe der Molprozente der Dicarbonsäure, die keine Terephthalsäure oder dessen esterbildendes Äquivalent ist, und des Diols mit einem niedrigen Molekulargewicht, das kein 1,4-Butandiol oder dessen esterbildendes Äquivalent ist, höchstens 25 beträgt, wobei der Copolyetherester zu 45 - 95 Gew% aus kurzkettigen Estereinheiten besteht, die Flüssigkeitsdurchlässigkeit von maximal 0,02 g/cm²/Std/23°C/0,07 bar und im Copolyetherester der Gewichtsanteil der langkettigen Estereinheiten 5 - 55% beträgt und das mittlere Kohlenstoff/Sauerstoff-Verhältnis der langkettigen Glycole zwischen 2,0 und 3,7 liegt und dessen Molekulargewicht 800 - 6000 beträgt mit der Maßgabe, daß, wenn das mittlere Atomverhältnis Kohlenstoff zu Sauerstoff der langkettigen Glycole zwischen 2,0 und 2,4 liegt, der Gewichtsanteil der langkettigen Estereinheiten 5 - 35% beträgt, und die Copolyetherester-Folie durch Flach- und/oder Blasextrusion aus Polymerschnitzeln, die in Wasser von 23°C eine Wasseraufnahme von höchstens 15 Gew%, bezogen auf das Trockengewicht der Schnitzel, aufweisen, hergestellt wurden.

Sowohl die Wasserdurchlässigkeit als auch die Wasserdampfdurchlässigkeit der Copolyetheresterfolien sind nicht nur von deren Zusammensetzung, sondern auch von ihrer Dicke abhängig. Bei jeder gewählten Foliendicke darf die Wasserdurchlässigkeit höchstens 0,02 g/cm²/Std/23°C/0,07 bar betragen, während die Wasserdampfdurchlässigkeit bei mindestens 500 g/m²/Tag/23°C/100-50 % rel. Luftfeuchtigkeit/Windgeschwindigkeit 0,3 m/s liegen muß. Es hat sich gezeigt, daß man bei Verwendung einer Polymerfolie mit einer Dicke zwischen 5 und 35 um sehr gute Ergebnisse erzielt. Ein optimales Ergebnis erhält man im allgemeinen mit einer Polymerfoliendicke zwischen 10 und 15 um.

Besonders bei dauernd bettlägerigen Patienten besteht die Gefahr des Wundliegens, so daß Druckstellen nach Möglichkeit vermieden werden müssen. Deshalb muß die Folie eine Geschmeidigkeit aufweisen, die eine problemlose Anpassung an die jeweilige Körperkontur ermöglicht. Folien mit einer

Dicke im genannten Bereich weisen eine Geschmeidigkeit auf, die für ein komfortables Liegegefühl ausreichend ist.

Wesentlich für gute Eigenschaften und hohen Komfort einer derartigen Matratzenauflage sind neben hoher Wasserdichtheit bei gleichzeitig hoher Wasserdampfdurchlässigkeit der Folie die Absorptionseigenschaften der Sauglage. Diese werden durch Flüssigkeitsaufnahme, Flüssigkeitsrückhaltung und Rückfeuchtung bestimmt:

Auf geeignete Materialien wird in der oben zitierten Europäischen Patentanmeldung verwiesen. Es handelt sich dabei um Polymere mit einem sehr hohen Wasseraufnahmevermögen, wie z.B. um modifizierte Cellulose und/oder Salze von vernetzten Polyacrylaten.

Derartige Produkte sind Einwegartikel, die nach dem Gebrauch verworfen werden. Sie werden vor allem dort eingesetzt, wo wiederverwertbare Sauglagen z.B. wegen nicht vorhandener chemischer Reinigungskapazität nicht eingesetzt werden können. Dies trifft beispielsweise für kleine Krankenhäuser oder für Arztpraxen zu.

In einer bevorzugten Ausführungsform besteht die Sauglage deshalb aus einem schweren Rauhgewebe aus Baumwolle, dem sog. Molton. Dieses Gewebe weist einen deckenden, stumpfen Rauhflor auf und ist häufig in Doppelschußtechnik hergestellt. Es besitzt die für die Sauglage geforderten Eigenschaften in ausreichendem Maße und weist den Vorteil der Wiederverwertbarkeit auf, da es auf einfache Weise in der Waschmaschine gewaschen oder chemisch gereinigt werden kann. Damit eignet es sich vorzüglich für den Gebrauch im häuslichen Bereich, sowie in Großkrankenhäusern und Kliniken mit hauseigener Wäscherei.

Durch die Wiederverwertbarkeit ergibt sich ein Kostenvorteil gegenüber den Einwegprodukten, da Lagerhaltungs- und Abfallbeseitigungskosten entfallen. Außerdem ist es möglich, die Moltonlage als Laken ohne weitere Decklage zu benutzen.

In einer weiteren Ausführungsform befindet sich zwischen der Matratze und der Folie eine weitere Schicht, die wasser- und damit auch wasserdampfdurchlässig ist. Sie kann als gewöhnliches Bettlaken ausgeführt sein. Dies wird zweckmäßigerweise dann der Fall sein, wenn Folie und Sauglage nicht die gesamte Oberfläche der Matratze überdecken. So kann es ausreichend sein, lediglich im mittleren Bereich der Matratze Folie und Sauglage unmittelbar auf dem Bettlaken anzubringen.

Auch ist es möglich, die Folie flächig mit einem Gewebe zu verbinden, das u.a. eine Art Stütz- oder Trägerfunktkion für die Folie übernimmt. Dieses Gewebe kann z.B. das Moltongewebe sein, was jedoch nicht in allen Fällen zweckmäßig ist, da aus hygienischen Gründen die Sauglage häufiger gereinigt werden sollte als die Folie. Deshalb wird die Folie bevorzugt auf ein zusätzliches Gewebe aufgebracht und mit ihm so verbunden, daß ein Laminat vorliegt.

In der vorgenannten Ausführungsform ist darauf zu achten, daß weder durch das zusätzliche Gewebe noch durch das Laminieren die Wasserdampfdurchlässigkeit nennenswert beeinträchtigt wird. Sinngemäß gilt dies auch für zusätzlich unmittelbar auf der Matratze angebrachte Bettlaken.

In der Praxis hat es sich als vorteilhaft erwiesen, die einzelnen Schichten der Matratzenauflage zu fixieren, um ein Verrutschen der Matratzenauflage als Ganzes oder der einzelnen Schichten gegeneinander durch die Bewegungen des Patienten auf der Matratze zu verhindern.

In einer bevorzugten Ausführungsform besteht die Matratzenauflage aus drei Schichten, nämlich einer Folie, einem Moltontuch sowie einem Bettlaken. Moltontuch und Bettlaken sind im Randbereich z.B. an zwei gegenüberliegenden Seiten miteinander verbunden, beispielsweise mittels einer Naht. Dazwischen befindet sich im eingeschobenen Zustand die Folie. Ein nennenswerts Verschieben der einzelnen Schichten gegeneinander durch Bewegungen des Patienten ist dadurch nicht mehr gegeben. Dennoch ist ein einfaches Trennen von Folie und den Geweben möglich. Dies ist von Bedeutung, wenn unterschiedliche Reinigungs- bzw. Waschvorschriften für die Folie und die Gewebe getrennte Behandlungen erfordern.

Zusätzlich ist die Matratzenauflage als Ganzes auf der Matratze gesichert. Dies ist auf einfache Weise dadurch zu erreichen, daß die Matratzenauflage eine etwas größere Breite aufweist als die Matratze selbst, so daß sie jeweils im Seitenbereich der Matratze mit einer um die Unterseite der Matratze herumgeführten Unterlage überlappt. Die Unterlage kann dabei aus einem einfachen Bettlaken bestehen. Im Überlappungsbereich sind Matratzenauflage und Unterlage mit lösbaren Befestigungsmitteln miteinander verbunden. Als Befestigungsmittel eignen sich besonders Klettbänder, Druckknöpfe oder Knopf/Knopflochpaarungen. Es ist auch möglich, als Unterlage Bänder oder Riemen auszuwählen, die ebenfalls lösbare Befestigungsmittel aufweisen. Damit kann auf einfache Weise ein Austausch der Matratzenauflage erfolgen, ohne daß die Matratze selbst angehoben werden muß.

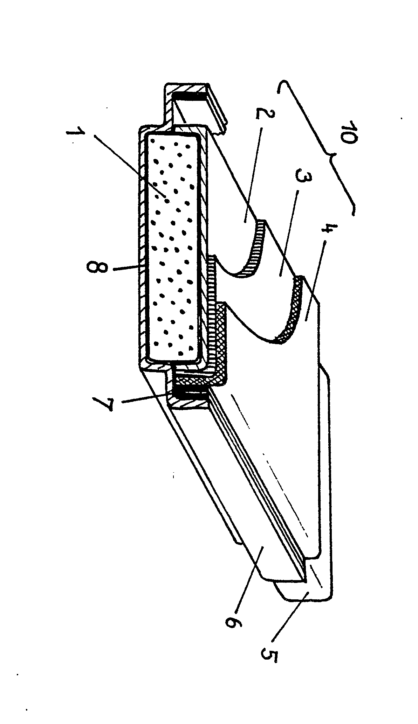

Die Erfindung wird weiter anhand der Figur erläutert. Die Figur zeigt schematisch in Schnittdarstellung den Aufbau der Matratzenauflage 10.

Unmittelbar auf der Matratze 1 befindet sich die wasser- und wasserdampfdurchlässige Schicht 2, z.B. in Form eines Bettlakens. Darüber ist die wasserundurchlässige und wasserdampfdurchlässige Folie 3 und darüber die flüssigkeitsaufnehmende Sauglage 4 angeordnet.

Die Matratzenauflage überlappt im Seitenbereich 5 mit der Unterlage 6 und ist mit ihr mittels eines Klettbandes 7 verbunden, wobei die Unterlage 6 um die Unterseite 8 der Matratze herumgeführt ist. Hier nicht dargestellt ist die beschriebene Verbindung der einzelnen Schichten untereinander.

Die Erfindung wird weiter anhand des nachstehenden Beispiels näher erläutert, das die Herstellung der erfindugsgemäßen Matratzenauflage beschreibt:
Es werden folgende Teile benötigt:
1 Folie 102 cm x 92 cm
- 2 steife Baumwolltücher 110 cm x 93 cm
- 1 Moltontuch 100 cm x 90 cm
- 4 m Schrägband von 2 cm Breite
- 2 x 92 cm Klettband
- Stecknadeln oder Druckknöpfe


Verarbeitung:


Eines der beiden Baumwolltücher wird als Unterlage verarbeitet. Hierzu wird je ein kleiner Saum an Kopf- und Fußende angebracht. An beiden Seitenenden werden jeweils feste Säume von 2,5 cm Breite genäht und darauf jeweils eine Klettbandseite angebracht.

Für die Herstellung der eigentlichen Matratzenauflage wird zunächst das zweite Baumwolltuch ebenfalls an Kopf- und Fußende mit einem kleinen Saum versehen. Darauf wird das Moltontuch gelegt und an beiden Seitenenden mit den Seitensäumen vernäht. Auf den Seitensäumen werden die Gegenseiten der Klettbänder angebracht.

Nun wird die Folie zur Verstärkung an allen Seiten 0,5 cm umgeschlagen und rundum mit dem Schrägband vernäht.

Schließlich wird die Folie zwischen Moltontuch und Baumwolltuch eingeschoben und erforderlichenfalls an den Ecken und ggf. an weiteren Punkten mit den Sicherheitsnadeln fixiert, die durch das Schrägband zu stecken sind. Es ist auch möglich, anstelle der Stecknadeln Druckknöpfe für die Fixierung vorzusehen.

Die angegegebenen Maße beziehen sich auf eine Matratzenauflage, die lediglich einen Teilbereich der Matratze überdecken. Bei anderen Ausführungsformen sind deshalb die angegebenen Maße entsprechend zu variieren.

der Matratze überdeckt.

## Ansprüche

1. Saugfähige Matratzenauflage, bestehend aus wenigstens zwei Schichten, von denen die erste, der Matratze zugewandte Schicht im wesentlichen flüssigkeitsundurchlässig ist und die darüberliegende zweite Schicht als flüssigkeitsaufnehmende Sauglage ausgebildet ist, dadurch gekennzeichnet, daß die im wesentlichen flüssigkeitsundurchlässige Schicht als Folie mit hoher Wasserdampfdurchlässigkeit ausgebildet ist.

2. Matratzenauflage nach Anspruch 1, dadurch gekennzeichnet, daß die flüssigkeitsaufnehmende Sauglage ein schweres Rauhgewebe aus Bauwolle ist.

3. Matratzenauflage nach Anspruch 1 oder 2, dadurch gekennzeichnet, daß eine weitere Schicht, die flüssigkeitsdurchlässig ist, unterhalb der flüssigkeitsundurchlässigen Schicht angeordnet ist.

4. Matratzenauflage nach Anspruch 3, dadurch gekennzeichnet, daß wenigstens zwei der drei Schichten zumindest an einem Abschnitt im Randbereich miteinander verbunden sind.

5. Matratzenauflage nach Anspruch 3, dadurch gekennzeichnet, daß die flüssigkeitsdurchlässige und die flüssigkeitsundurchlässige Schicht flächig miteinander verbunden sind.

6. Matratzenauflage nach einem der Ansprüche 1 bis 5, dadurch gekennzeichnet, daß sie eine größere Breite aufweist als die Matratze, so daß sie jeweils im Seitenbereich der Matratze mit einer um die Unterseite der Matratze herumgeführten Unterlage überlappt und mit der Unterlage im Überlappungsbereich mit lösbaren Befestigungsmitteln verbunden ist.

7. Matratzenauflage nach Anspruch 6, dadurch gekennzeichnet, daß die Befestigungsmittel Klettbänder, Druckknöpfe oder Knopf/Knopflochpaarungen sind.

8. Matratzenauflage nach einem der Ansprüche 1 bis 7, dadurch gekennzeichnet, daß wenigstens eine der Schichten nur einen Teil der Gesamtlänge